# EUROPEAN PATENT SPECIFICATION

(11) **EP 2 511 336 B1**
(45) Date of publication and mention of the grant of the patent: **23.11.2016**
(21) Application number: 12164098.1
(22) Date of filing: 13.04.2012
(51) Int. Cl.: C08L 29/04, A61L 31/04, A61L 31/12, C09B 11/24

(54) **THIXOTROPIC COMPOSITION, ESPECIALLY FOR POSTOPERATIVE ADHESION PROPHYLAXIS**
THIXOTROPIEZUSAMMENSETZUNGEN, INSBESONDERE ZUR POSTOPERATIVEN ADHÄSIONSPROPHYLAXE
COMPOSITION THIXOTROPE, NOTAMMENT POUR PRÉVENIR L'ADHÉRENCE POSTOPÉRATOIRE

(30) Priority: 15.04.2011 DE 102011007528
(43) Date of publication of application: 17.10.2012
(73) Proprietor: Aesculap AG, 78532 Tuttlingen/Donau (DE)
(72) Inventor: Bargon, Rainer, 78532 Tuttlingen (DE); Odermatt, Erich, 8200 Schaffhausen (CH)
(74) Representative: Patentanwälte Ruff, Wilhelm, Beier, Dauster & Partner mbB

(56) References cited:
- S. CHETAN KUMAR ET AL.: "Formulation and Evaluation of Chitosan-Gellan Based Methotrexate Implants", JOURNAL OF MACROMOLECULAR SCIENCE, PART A: PURE AND APPLIED CHEMISTRY, vol. 45, no. 8, 2008, pages 643-649, XP8153206,
- LI, JING; KAMATH, KALPANA; DWIVEDI, CHANDRADHAR: "Gellan film as an implant for insulin delivery", JOURNAL OF BIOMATERIALS APPLICATIONS, vol. 15, no. 4, 2001, pages 321-343, XP8153244, DOI: 10.1106/R3TF-PT7W-DWN0-1RBL
- BERND MARTIN JAENIGEN ET AL: "The new adhesion prophylaxis membrane A-part -From in vitro testing to first in vivo results", JOURNAL OF BIOMEDICAL MATERIALS RESEARCH PART B: APPLIED BIOMATERIALS, vol. 89B, no. 2, 1 May 2009 (2009-05-01), pages 293-299, XP55032037, ISSN: 1552-4973, DOI: 10.1002/jbm.b.31215

## Description

The present invention relates to a thixotropic composition useful for prophylaxis of postoperative adhesions in particular, a receptacle containing the composition, an applicator containing the composition and also a method of making the composition.

The manifestation of undesired tissue adhesions following surgical interventions is a well-known problem in surgical care (Treutner KH, Schumpelick V. Adhäsionsprophylaxe: Wünsche und Wirklichkeit. Chirurg 2000; 71:510-517).

Tissue adhesions are generally the formation of strands of fibrin between mutually opposite, damaged tissue structures. Adhesions can arise in the abdominal cavity, the pelvic cavity, especially in the region of the internal female genitalia, at the pericardial sac and also at injured/damaged nerves.

Such tissue adhesions can have very serious consequences for the afflicted. Mechanical ileus is adhesion related in the majority of cases and has a 10% mortality rate (Menzies D, Parker M, Hoare R, Knight A. Small bowel obstruction due to postoperative adhesions: treatment patterns and associated costs in 110 hospital admissions. Ann. R. Coll. Surg. Engl. 2001; 83:40-46). Furthermore, the occurrence of postoperative adhesions is thought to account for those 20% of women who are infertile after an operation (Gomel V. Reproductive surgery. Minerva Ginecol (abbr) 2005; 57(1):21-28). In paediatric heart surgery, adhesions between the epicardium and the pericardium are frequently fatal because resternotomy may be needed in these cases and it entails a high risk of damage to the large heart vessels of the children in question (Lodge AJ, Wells WJ, Bacher CL, O'Brien JE, Erie HA, Bacha EA, Yeh T, DeCampli WM, lavin PT, Weinstein S. Novel Bioresorbable Film Reduces postoperative Adhesions After Infant Cardiac Surgery. Ann. Thorac. Surg. 2008; 86:614-621).

Against this background, there are now various treatment approaches to ideally avoid the occurrence of postoperative adhesions.

One treatment approach consists in administering non-steroidal antiinflammatory drugs or corticosteroids in order to intervene directly in the enzymatic coagulation cascade. Disadvantages with this are the dependence of a successful outcome on the administered dose, a high risk of bleeding and also disrupted wound healing.

A further treatment approach involves the administration of liquids (instillation) in order to forestall the formation of undesired tissue adhesions by hydroflotation.

However, the majority of treatment approaches is now based on the use of membranes and gels which function as mechanical barriers to adhesion and thereby keep adhesion-liable tissue structures separate from each other at least temporarily.

For instance, polyvinyl alcohol hydrogels (PVA hydrogels) with anti-adhesive properties for use in visceral surgery and gynaecology are known from the documents EP 1 384 450 A1, WO 02/09789 A2, EP 1 579 876 A2, EP 1 666 077 A2 and DE 10 2009 029 717 A1.

The use of PVA hydrogels for adhesion prophylaxis is also described in the non-patent literature (Lang RA, Weisgerber C, Grüntzig PM, Weis C, Odermatt EK, Kirschner MH. Polyvinyl Alcohol Gel Prevents Adhesion Re-Formation After Adhesiolysis in a Rabbit Model. J. Surg. Res. 2009; 153(1): 12-16).

US 5,284,897 relates to a thixotropic gel which, in addition to partially hydrolyzed polyvinyl alcohol, also includes a thickening agent selected from the group consisting of sodium carboxymethylcellulose, sodium alginate and xanthan.

EP 1 323 436 A1 discloses an adhesion barrier composition comprising sodium carboxymethylcellulose and gellan with or without, among other possible ingredients, polyvinyl alcohol.

US 5,318,780 discloses an aqueous liquid which can be gelled in situ and which is useful for avoiding postoperative adhesions in particular. The mixture includes an ionic polysaccharide, a film-forming polymer and also, optionally, a divalent metal salt which promotes the gelling of the polysaccharide and is present in excess relative to the polysaccharide. To avoid premature gelling, the liquid which can be gelled in situ preferably finds application as two-part system.

The rheological properties and also structural properties of gellan gels are also known from the scientific literature (Quigley et al.: Rheological evaluation of deacetylated gellan gum (Gelrite) for pharmaceutical use; International Journal of Pharmaceutics, 73 (1991) 117-123 and Quinn FX, Hatakeyama T, Yoshida H, Takahashi M. The Conformational Properties of Gellan Gum Hydrogels, Polymer Gels and Networks 1993, 1, 93-114).

A fundamental disadvantage of gels based mainly on PVA is that they display little if any adhesion/adherence to tissue. As a result, there is a risk that these gels will not stay in place on an operation site after application but will, generally as a result of physical movements of the patient, undergo an undesired dislocation.

True, the tissue adherence of PVA gels can in principle be improved through appropriate additization, for example with thickening agents. However, additizations of this kind frequently have an adverse effect on the final properties of the gels. For example, gels thus additized cannot be transparent or heat labile. A further problem can be that, as a consequence of additization, gel viscosity increases to such an extent that the gels can only be applied from an applicator under appreciable application of force.

Additional difficulties can arise because, to avoid premature gelling, gel components frequently have to be kept spatially separate from each other and the components are only mixed with each other in situ to obtain a gel. In addition to the risk of dislocation due to the time delay caused by the gelling process, these "in situ technologies" can also give rise to inhomogeneities which may be due to local concentration differences.

The use of gellan including formulations for the manufacture of methotrexate releasing implants and insulin delivering films is described in literature (Kumar at al.: "Formulation and Evaluation of Chitosan-Gellan Based Methotrexate Implants." Journal of Macromolecular Science Part A: Pure and Applied Chemistry (2008) 45, 643 - 649. Jing Li at al.: "Gellan Film as an Implant for Insulin Delivery". Journal of Biomaterials Applications Volume 15 no. 4, pages 321-343 - April 2001).

The present invention therefore has for its object to provide a composition useful for prophylaxis of unwanted tissue adhesions in particular which evades the prior art difficulties. The composition shall more particularly be easy to apply and, after application, shall ideally remain in location on an operation site.

This object is achieved according to the present invention by a composition having the features of independent claim 1. Preferred embodiments of the composition are subject matter of dependent claims 2 to 13. Finally, the invention relates to the production process having the features of claim 14.

The composition according to the present invention is a thixotropic composition, preferably in the form of a hydrogel, comprising a film-forming polymer, gellan and/or agar, a physiologically compatible metal salt and water.

A thixotropic composition for the purposes of the present invention is a composition which liquefies on exposure to mechanical forces, especially shearing forces as can arise for example in stirring, shaking and/or in delivery from an applicator, for example through actuation of a delivery piston or plunger, and resolidifies as the mechanical stress ends, preferably by gelling. The viscosity increase or decrease when the mechanical stress starts and ends, respectively, can take place with a certain time delay, i.e. a so-called hysteresis. This time delay can be in the range from a few milliseconds up to several minutes.

A film-forming polymer for the purposes of the present invention is a polymer with film-forming properties, especially on adding a solvent such as water for example. Polyvinyl alcohol (PVA) is a particularly preferred film-forming polymer in the present invention.

The term "gellan" for the purposes of the present invention comprehends not just the neutral heteropolysaccharide, but especially also its salts. Examples of such salts can be sodium, potassium, magnesium, calcium and/or strontium salts. According to the present invention, it is therefore the case that even mixed salts of gellan can be comprehended by the present invention.

The term "agar" for the purposes of the present invention comprehends not just the mutual heteropolysaccharide, but especially also its salts. Examples of such salts can be sodium, potassium, magnesium and/or calcium salts. According to the present invention, it is therefore the case that even mixed salts of agar can be comprehended by the present invention.

A physiologically compatible metal salt for the purposes of the present invention is a salt which is medically safe to administer to a patient, i.e. causes no undesired or else medically negligible effects after its administration.

The present invention rests on the surprising finding that a composition, preferably a hydrogel, having thixotropic and in particular anti-adhesive properties is obtainable by starting with the components: film-forming polymer, which is polyvinyl alcohol for preference, gellan and/or agar, a physiologically compatible metal salt and water. Among these components, the gellan and/or agar act as thixotropic agents, preferably together with the metal salt. The gellan and/or agar can more particularly be regarded as thixotropic thickeners.

The composition according to the present invention is particularly advantageous because it takes the form of a firm/dimensionally stable composition, more particularly a firm/dimensionally stable hydrogel, at room temperature and under atmospheric pressure, especially under standard conditions, without mechanical stress and especially without shearing stress. However, once mechanical forces, in particular shearing forces, come to act on the composition, its viscosity decreases and the composition becomes liquid as a result. Since such forces arise for example in the course of the composition being delivered from an applicator by actuating a delivery plunger or piston, application of the composition according to the present invention to an operation site is simple and without complications. On removing the mechanical stress, for example after application has taken place, the viscosity increases again and the composition solidifies, which distinctly reduces the risk of the composition dislocating.

In one preferred embodiment, the composition at room temperature (22°C) and under atmospheric pressure, especially under standard conditions, has a complex viscosity, the amplitude of which is at least twice as high as the shear viscosity of the composition at room temperature (22°C) and under atmospheric pressure, especially under standard conditions. Initial viscosity for the purposes of the present invention is to be understood as meaning the composition's complex viscosity before the first application of a mechanical stress such as a shearing stress for example. Shear viscosity for the purposes of the present invention, by contrast, is to be understood as meaning the composition's viscosity under application of constant shearing forces in the course of a circular movement at constant frequency. Shear viscosity of this type can be determined for example using a rheometer with a plate-cone arrangement where the composition is generally positioned between the cone and the plate and the cone describes circular movements or rotating movements to exert shearing forces on the composition.

"Complex viscosity" for the purposes of the present invention is to be understood as meaning the sum of the elasticity modulus (or storage modulus) G' and the loss modulus G". The elasticity/storage modulus G' reflects the stored mechanical energy. The loss modulus G" reflects the dissipatively, i.e. unrecoverably, released viscous proportion of the energy introduced into the composition.

Complex viscosity is generally determined using the so-called oscillatory measurement. In it, measurements are carried out under oscillatory stress (vibration measurement). To this end, the in-test material is usually exposed to a sinusoidal deformation at small amplitude. Complex viscosity is generally proportional to the phase-retarded, oscillatory resiling force of the composition and especially of the hydrogel. The small amplitude ensures measurement in the linear viscoelastic region. The oscillatory measurement to determine the complex viscosity of a material is known per se to a person skilled in the art and so no further explanations are needed here.

In one preferred embodiment, the composition at room temperature (22°C) and under atmospheric pressure, especially under standard conditions, has an initial viscosity between 10 and 100 Pas, especially 10 and 40 Pas and preferably 15 and 25 Pas.

In a further preferred embodiment, the composition at room temperature (22°C) and under atmospheric pressure, especially under standard conditions, and at a shear stress (σ) of 50 Pa to 100 Pa has a shear viscosity between 25 and 100 Pas. At a shear stress (σ) of 70 Pa, the composition preferably has a shear viscosity of 70 Pas.

In a further embodiment, the composition in a non-steam-sterilized state at room temperature (22°C) and under atmospheric pressure, especially under standard conditions, has a complex viscosity which is greater especially by a factor of 2 than the amplitude of the complex viscosity of the composition after steam sterilization.

The composition in a further embodiment at room temperature (22°C) and under atmospheric pressure, especially under standard conditions, and under the influence of a shearing stress and preferably under the influence of a shearing force of 70 Pa has a shear viscosity of 20 Pas before steam sterilization and of 7 Pas after steam sterilization at 200 Pa.

The composition according to the present invention has a semipermeable barrier structure in preferred embodiments. The composition represents a preferably impenetrable barrier to strands of fibrin and/or of fibrinogen. Yet the composition can be permeable to, in particular, low molecular weight compounds, molecules and the like and/or to salts having small ionic radii. As a result, there can be an exchange of nutrients which is beneficial to wound healing through the composition and/or a release of active ingredients which is beneficial to wound healing from the composition.

In one preferred embodiment, the composition includes a film-forming polymer, gellan, a physiologically compatible metal salt and water.

The composition in a further embodiment includes the film-forming polymer at between 0.1 and 30 wt%, especially 1 and 15 wt% and preferably 5 and 10 wt%, based on overall composition weight.

The film-forming polymer is preferably a film-forming polymer which is water soluble.

The film-forming polymer may be selected for example from the group consisting of alkylcelluloses such as for example methylcellulose and/or ethylcellulose, hydroxyalkylcelluloses such as for example hydroxyethylcellulose and/or hydroxypropylcellulose, hyaluronic acid, chondroitin sulphate, dextrins, polydextrins, cyclodextrins, maltodextrin, polydextrose, pectins, collagen, gelatin, acrylamide polymers, acrylic acid polymers, methyl methacrylate polymers, 2-hydroxyethyl methacrylate polymers, polyvinyl alcohol (PVA), polyvinylpyrrolidone, polyethylene glycols, polyethylene oxide, salts thereof, copolymers thereof and mixtures thereof. A copolymer for the purposes of the present invention is a polymer which is composed of two or more different units of monomer.

In one advanced embodiment, the film-forming polymer is not a polysaccharide.

The film-forming polymer is preferably selected from the group consisting of polyvinyl alcohol, polyethylene glycols, polyethylene oxide, salts thereof, copolymers thereof and mixtures thereof. The polyethylene glycols may be in particular branched, dendrimerically constructed, terminally functionalized and/or modified/capped polyethylene glycols. Concerning suitable polyethylene glycols, reference is made for example to US 5,874,500, the disclosure content of which is incorporated herein by express reference.

As already mentioned more than once, it is particularly preferable according to the present invention for the film-forming polymer to be polyvinyl alcohol (PVA).

In a further, particularly preferred embodiment, the composition includes polyvinyl alcohol, gellan, a physiologically compatible metal salt and water.

The average molecular weight of the polyvinyl alcohol is preferably between 100 000 and 400 000 daltons, especially 100 000 and 300 000 daltons and more preferably 100 000 and 150 000 daltons.

The polyvinyl alcohol may further be in a partially - only partially in particular - acetylated state. The polyvinyl alcohol's degree of acetylation may be for example < 10%, especially < 4% and preferably < 2%, based on the total number of hydroxyl groups in the polyvinyl alcohol.

In principle, the polyvinyl alcohol can also be in a partially hydrolyzed state. However, it is preferable according to the present invention for the polyvinyl alcohol to be in an unhydrolyzed/non-hydrolyzed state.

The gellan provided according to the present invention is typically a heteropolysaccharide which is composed of linear tetrasaccharide units, while the tetrasaccharide units each include 1,3-β-D-glucose, 1,4-β-D-glucuronic acid, 1,4-β-D-glucose and 1,4-α-L-rhamnose, which may be each esterified with acetic acid and glyceric acid. The gellan provided according to the present invention is preferably, in particular typically, prepared via fermentation using the bacterial strain Pseudomonas elodea. Gellans suitable according to the present invention are commercially available for example under the designations KELCOGEL^{®}, KELCOGEL AFT, KELCOGEL CG-LA, KELCOGEL CG-HA and KELCOGEL LT100. Processes for preparing gellan are described for example in the documents US 4,326,052 and US 4,326,053, the disclosure content of which is incorporated herein by reference. The material KELCOGEL CG-LA has no acetyl groups, but does have a glycerate unit per tetrasaccharide unit. KELCOGEL CG-HA additionally has an acetyl group per every second tetrasaccharide unit.

The gellan is with particular advantage thermally stable and therefore makes a net contribution to improving the thermal stability of the composition according to the present invention. More particularly, the gellan is still stable at temperatures typically used for performing steam sterilizations.

The agar provided as an alternative or addition to the gellan is a heteropolysaccharide constructed of agarose and agaropectin. Agar is typcially isolated from the cell wall of red algae. Agarose is the main constituent of agar. Agarose is a linear construction of alternating D-galactose and 3,6-anhydro-L-galactose units. Agaropectin consists of partly sulphated L-galactose units linked together in linear fashion. It also contains 3,6-anhydro-L-galactose as well as the corresponding uronic acids.

The gellan and/or agar, preferably gellan, in a further preferred embodiment has an average molecular weight between 100 000 and 1 000 000 daltons, especially 200 000 and 700 000 daltons and preferably 400 000 and 600 000 daltons.

The composition according to the present invention includes gellan and/or agar, especially gellan, at between 0.2 and 0.4 wt%, based on the overall composition weight. The gellan and/or agar percentages described in this section are particularly advantageous for forming the thixotropic properties of the composition.

In a further embodiment, the gellan and/or agar, preferably gellan, is in a partially - only partially in particular - deacetylated state. The gellan may for example have a degree of acetylation < 10 mol%, based on a tetrasaccharide unit of the gellan.

The metal ions of the metal salt provided according to the present invention preferably form coordinative bonds with negatively charged carboxylate groups of gellan and/or agar. The result is a preferably firm or viscid compound structure which preferably also has elastic properties.

The physiologically compatible metal salt provided according to the present invention may in principle be one specific metal salt or else a mixture of various metal salts.

The physiologically compatible metal salt may further be in principle a salt of a monovalent or polyvalent, in particular di- and/or trivalent, metal. However, salts of divalent metals are particularly preferable in the present invention. This is because divalent ions of metals have been found to form a particularly strong coordinative bond with the carboxylate groups in gellan and/or agar.

In an advanced embodiment, the physiologically compatible metal salt is an inorganic salt, especially an alkali metal salt, an alkaline earth metal salt and/or a transition group metal salt such as zinc salts for example.

It is preferable for the physiologically compatible metal salt to be an alkaline earth metal salt, especially an alkaline earth metal halide and preferably an alkaline earth metal chloride.

The physiologically compatible metal salt may be selected for example from the group consisting of sodium chloride, potassium chloride, magnesium chloride, calcium chloride, strontium chloride, zinc chloride and mixtures thereof. Calcium chloride is particularly preferable in the present invention.

In a further embodiment, the composition has a larger gellan and/or agar content, preferably gellan content, than metal salt content. For example, the composition may have a weight ratio (wt%) of gellan and/or agar, preferably gellan, to the physiologically compatible metal salt in the range from 50:1 to 1:1, especially in the range from 50:1 to 2:1, preferably in the range from 25:1 to 2:1 and more preferably in the range from 10:1 to 5:1.

The composition in a further embodiment includes the physiologically compatible metal salt at not more than 1 wt%, based on overall composition weight. Particularly preferably, the proportion of metal salt in the composition is between 0.01 and 1.0 wt%, especially 0.01 and 0.1 wt% and more preferably 0.02 and 0.07 wt%, based on overall composition weight. Further preferably, the proportion of metal salt in the composition is between 0.05 and 0.1 wt%, based on overall composition weight. The metal salt percentages described in this section contribute to a particular degree to the fact that the composition according to the present invention is a dimensionally stable composition, especially a dimensionally stable hydrogel, when kept at room temperature and under atmospheric pressure, especially under standard conditions, but readily liquefies on exposure to the influence of mechanical forces, in particular shearing forces, and resolidifies on removing the forces.

In a further embodiment, the composition includes water at between 50 and 98 wt%, especially 60 and 98 wt% and preferably 65 and 98 wt%, based on overall composition weight.

It may be further provided according to the present invention for the composition to include one or more admixture agents. Examples of suitable admixture agents will now be more particularly described.

It is further preferable for the composition to include a tissue adhesive to improve the tackiness with regard to, or the adherence to, biological, especially human and/or animal, tissues. This may additionally serve to minimize the risk of undesired dislocation of the composition from an operation site.

The tissue adhesive may be selected from the group consisting of alkylcelluloses, hydroxyalkylcelluloses, carboxyalkylcelluloses, salts thereof and mixtures thereof. It is preferable for the tissue adhesive to be a carboxyalkylcellulose or a salt, especially alkali metal salt and preferably sodium salt, thereof.

In an advanced embodiment, the tissue adhesive is selected from the group consisting of methylcellulose, hydroxymethylcellulose, hydroxyethylcellulose, hydroxypropylcellulose, methylhydroxypropylcellulose, cellulose sulphates, dextrins, acetylstarch, starch phosphates, carboxymethylstarch, hydroxyethylstarch, hydroxypropylstarch, salts thereof and mixtures thereof. Preferably, the tissue adhesive is carboxymethylcellulose or a salt thereof, preferably sodium carboxymethylcellulose.

In a further embodiment, the tissue adhesive is present in the composition at between 0.01 and 0.5 wt%, especially 0.01 and 0.49 wt% and preferably 0.1 and 0.4 wt%, and more preferably 0.3 and 0.4 wt%, based on overall composition weight.

In an advantageous embodiment, the composition additionally includes a dye. The addition of a dye makes it possible for example for the surgeon to visually police the application of the composition to an operation site. In addition, a coloured composition makes it possible for the occurrence of adhesions to be inspected as part of a reoperation where the composition according to the present invention is used as adhesion barrier. In a reoperation, moreover, the efficacy of the composition as adhesion barrier can be visually inspected and any dislocation of the composition visually quantified.

The dye may be selected for example from the group consisting of FD & C Blue No.1, FD & C Green No.3, FD & C Red No.3, FD & C Yellow No.5, FD & C Yellow No.6, FD & C Blue No.2, FD & C Red No.40, FD & C Green No.5, D & C Violet No.2, D & C Green No.5, D & C Red No.6, D & C Red No.7, D & C Red No.21, D & C Red No.22, D & C Red No.27, D & C Red No.28, D & C Red No. 30 and mixtures thereof.

The dye may further be present in the composition at between 0.001 and 0.05 wt%, especially 0.01 and 0.05 wt% and preferably 0.01 and 0.025 wt%, based on overall composition weight.

It may further be provided according to the present invention for the composition to include an anti-freeze. This is a particularly advantageous way for any solidification, especially gelling, of the composition at low temperatures (cryogelling) to be prevented or at least distinctly weakened. This in turn means improved stability for the composition in relation to a possibly sudden temperature drop and also improves the long-term storability of the composition at low temperatures.

The anti-freeze is preferably selected from the group consisting of isopropanol, propanediol and mixtures thereof.

The composition may further include the anti-freeze at between 1 and 20 wt%, especially 5 and 15 wt%, and preferably 7 and 13 wt%, based on overall composition weight.

In a further embodiment, the composition includes active ingredients and/or is additized with active ingredients. The active ingredients are preferably biologically, pharmaceutically, medically and/or cosmetically active ingredients. The active ingredients may more particularly be selected from the group consisting of antimicrobially, especially antibiotically, active ingredients, disinfectingly active ingredients, antiinflammatorially active ingredients, analgesically active ingredients, cellular growth factors, cellular differentiation factors, cellular adhesion factors, cellular recruitment factors, cytokines, peptides, proteins such as for example collagen, lipids, polysaccharides such as for example hyaluronic acid, oligonucleotides, polynucleotides, DNA, RNA, salts thereof and mixtures thereof.

It may therefore be entirely preferable according to the present invention for the thixotropic composition of the present invention to be used as a vehicle/carrier for active ingredients and/or for delivery of active ingredients (drug delivery system).

In a particularly preferred embodiment, the composition is composed of the following components:
a) 0.1 to 30 wt% of film-forming polymer, preferably polyvinyl alcohol,
b) 0.2 to 0.4 wt% of gellan and/or agar, preferably gellan,
c) 0.01 to 1 wt% of a physiologically compatible metal salt,
d) 0.01 to 0.5 wt%, especially 0.01 to 0.49 wt%, of a tissue adhesive,
e) 50 to 98 wt% of water, and optionally
f) 1 to 20 wt% of one or more admixture agents, especially to inhibit cryogelling of the composition, especially of the film-forming polymer.

With regard to further features and advantages of the components mentioned in the last or above paragraph, the description heretofore and hereinafter is referenced in its entirety.

In a further embodiment of the invention, the composition consists of the film-forming polymer, gellan and/or agar, the physiologically compatible metal salt, water and optionally one or more admixture agents. The admixture agent(s) are preferably selected from the group consisting of tissue adhesive, dye, anti-freeze, active ingredients and combinations thereof. With regard to possible admixture agents, reference is made particularly to the admixture agents described in the preceding embodiments (tissue adhesive, dye, anti-freeze and active ingredients) and also their properties and features.

In principle, the film-forming polymer, the gellan and/or agar, the physiologically compatible metal salt, water and also any of the optional admixture agents present can be present, at least in part, spatially/physically separated from one another.

However, it is particularly preferable in the present invention for the composition to be in the form of a mixture, in particular a ready-to-use mixture. In other words, it is preferable when the film-forming polymer, the gellan and/or agar, the physiologically compatible metal salt, water and also any of the optional admixture agents present, especially selected from the group consisting of tissue adhesive, dye, anti-freeze, active ingredients and combinations thereof, are present in the composition neither spatially nor physically separated from one another. For example, the composition may be present as a mixture in a receptacle of an applicator, especially in a syringe barrel.

The composition in an advantageous embodiment has a gel point above 95°C. The gel point for the purposes of the present invention is the temperature or temperature range at or in which the composition according to the present invention transitions from the liquid state into a firm, especially gel-like, state.

In a further embodiment, the composition is transparent. A transparent composition is decisively advantageous for the surgeon since they are able to look through a transparent composition on the operation site to be treated and see the tissue structures underneath, which is particularly advantageous with regard to early detection of bleeding and/or any dislocation of the composition.

The composition in a further advantageous embodiment is in a steamsterilized state. In other words, the composition according to the present invention may have been sterilized with steam. Steam sterilization has the advantage that any inhomogeneities can be eliminated to obtain a homogeneous or essentially homogeneous composition. Inhomogeneities of this type may be attributable for example to local concentration differences of components, for example the physiologically compatible metal salt, in the composition. Inhomogeneities of this type can arise during a transfer operation for example.

The composition according to the present invention may in principle be configured as film, membrane, sponge or gel, preferably hydrogel. As mentioned at the beginning, the composition is preferably in the form of a thixotropic hydrogel.

The thixotropic composition of the present invention can be used in general surgical care. Preferably, however, the composition is used in abdominal surgery, visceral surgery, paediatric cardiac surgery and/or surgical gynaecology.

The composition is preferably used for prophylaxis of postoperative tissue adhesions. In other words, the composition preferably serves as adhesion prophylaxis composition. For example, the composition can be used to avoid tissue adhesions in the abdominal cavity, in the pelvic cavity, especially in the region of the internal female genitalia, on the pericardial sac and/or in relation to damaged nerves.

Further, it is disclosed a receptacle containing the thixotropic composition of the present invention. The receptacle is preferably a receptacle of an applicator. For example, the receptacle may be configured as cartridge or syringe barrel. To avoid unnecessary repetition, the description heretofore and hereinafter is referenced with regard to further features and advantages of the composition.

Furthermore, it is disclosed an applicator containing the thixotropic composition of the present invention. The applicator may be for example in the form of a syringe, preferably a single-chamber syringe. To avoid unnecessary repetition, the description heretofore and hereinafter is referenced with regard to further features and advantages of the composition.

A further aspect of the present invention relates to a process for preparing the thixotropic composition of the present invention, wherein a film-forming polymer, gellan and/or agar, a physiologically compatible metal salt, water and optionally one or more admixture agents, especially selected from the group consisting of tissue adhesive, dye, anti-freeze, active ingredients and combinations thereof, are mixed with one another, preferably to obtain a thixotropic hydrogel.

In one preferred embodiment, a solution of the film-forming polymer and water is initially prepared. It is preferable for this for a mixture of the film-forming polymer and water to be warmed/heated, especially to a temperature range between 70 and 90°C. To obtain such a solution, the film-forming polymer and the water can be stirred together for several hours, for example for two hours.

It is further preferable in the present invention for the solution of the film-forming polymer and water to be subsequently admixed with the gellan and/or agar, the physiologically compatible metal salt and also optionally the admixture agent(s). These components are preferably added to the solution of the film-forming polymer in water while it is still hot. The mixture thus obtained can then be further stirred for a certain period, for an hour for example, before it is transferred into a suitable applicator for example.

In one advantageous embodiment, the mixture of the film-forming polymer, gellan and/or agar, the physiologically compatible metal salt, water and also optionally the admixture agent or agents is subjected to a steam sterilization. Typically, the steam sterilization is carried out in a dedicated steam autoclave at 121°C for not less than 30 minutes and not more than 60 minutes.

It is particularly preferable to use polyvinyl alcohol (PVA) as film-forming polymer.

With regard to further features and advantages of the process, especially the composition and/or its components, the description heretofore and hereinafter is referenced in full.

It is finally disclosed the use of a film-forming polymer, of gellan and/or agar, of a physiologically compatible metal salt, of water and optionally of one or more admixture agents to prepare a thixotropic composition, preferably a thixotropic hydrogel, especially for adhesion prophylaxis, i.e. for avoiding postoperative tissue adhesions. The film-forming polymer is preferably polyvinyl alcohol (PVA). With regard to further features and advantages, in particular concerning the components mentioned in this section, the description heretofore and hereinafter is referenced in full.

At this point, the advantages of the composition according to the present invention, which is preferably in the form of a thix
otropic hydrogel, will be summarized once more as follows:
- The composition according to the present invention has thixotropic properties. This is achieved in particular as a result of the gellan and/or agar in the composition acting as thixotroping agents, especially in combination with the metal ions of the physiologically compatible metal salt.
- The composition preferably has a high starting/initial viscosity (complex viscosity) and a, by comparison therewith, distinctly reduced shear viscosity. As a result, the composition can be rendered thinly liquid or flowable by exposing it to the agency of a shearing force, of the type typically arising on delivery from a suitable applicator, for example as a result of actuating a delivery plunger or piston. This enables accurately targeted and, more particularly, uncomplicated application of the composition to an operation site. After application, the viscosity of the composition generally goes rapidly back up as a result of it no longer being subject to the agency of a shearing force, and the composition solidifies, preferably by gelling. Fixed application of the composition to an operation site becomes possible in effect as a result. The risk of the composition dislocating due to physical movements on the part of the patient in particular, for example as a result of intestinal peristalsis, is significantly lower.
- The composition has the particular advantage of anti-adhesive properties, especially with regard to fibrinous tissue adhesions.
- The composition is preferably in the form of a directly ready-to-use, i.e. directly administrable mixture, preferably hydrogel. Technically inconvenient and, more particularly, malfunction-prone in situ technologies can be circumvented as a result.
- The composition is preferably further notable for a certain thermal stability whereby temperature fluctuations occurring during storage and/or transportation can be more efficiently absorbed without the properties of the composition being adversely affected as a result. The use of suitable anti-freezes, moreover, prevents or at least distinctly weakens any cryogelling of the composition, especially of the film-forming polymer.
- When a dye is (optionally) included in the composition, better visual application and localization control by the surgeon is possible, especially in relation to minimally invasive interventions. In addition, any dislocations of the composition are quicker to visually localize and especially visually quantify in the course of reoperations.

Further features and advantages of the invention will be apparent from the following description of preferred embodiments in the form of examples and also in combination with the features of dependent claims. In these embodiments, individual features of the invention can be actualized alone or in combination with other features. The preferred embodiments described are merely to be understood as a descriptive and certainly not in any way as a limiting disclosure.

### Example 1 : Preparing a thixotropic hydrogel

A reaction pot in an RT50 vacuum mix processing rig with Biotrona BA-C80 and a PX-RW 0.55 anchor stirrer from Kinematica (Switzerland) was charged with 27.3 kg of twice-distilled water from B. Braun (Ecobag^{®}) and 2.50 kg of polyvinyl alcohol (having an average molecular weight of 100 to 300 kD (kilodaltons) and a degree of acetylation < 2% with the trade name Mowiol^{®} 56-98 from Kuraray and sealed. This was followed by stirring at 70 to 90°C for two hours. The hot solution was then admixed with 100 g of sodium carboxymethylcellulose according to EP with the trade name Cekol 2000P (FIN) from Kelco, 105 g of deacetylated gellan according to EP (degree of acetylation < 4%) with the trade name KELCOGEL CG-LA^{®}, 50 g of a 30 wt% solution of calcium chloride p.a. from Merck in the abovementioned twice-distilled water and also 3.75 g of FD&C Green No. 3 from Goldmann. The mixture was stirred for a further hour. Thereafter, the solution at a mixture temperature of at least 60°C was transferred into syringes using a Netzsch metering pump. The hot hydrogel did not have to be filtered. The filled syringes were subsequently sterilized in an appropriate apparatus within steam-permeable secondary packaging in a steam autoclave at 121 °C for at least 30 minutes and at most 60 minutes, especially for 60 minutes.

### 1.1 Characterizing the hydrogel prepared as per Example 1

A Gemini 150 plate-cone Bohlin rheometer was used to determine the complex viscosity of the hydrogel using the so-called Searle method. The plate had a diameter of 40 mm and an angle of 4°. Gap size was 150 µm.

The complex viscosity of the hydrogel was 36 Pas in the non-steam-sterilized state and 21.2 Pas in the sterilized state.

The gel was subsequently subjected to a 20 minute pre-shear at 10 Hz cycle frequency (integration time: 2 s, delay time: 2 s and wait time: 2 s). Subsequent determination of the shear viscosity gave a value of 20 Pas. After steam sterilization was performed, the hydrogel was found to have a shear viscosity of 7.07 Pas.

A thixotropy test of the hydrogel prepared according to Example 1 was further carried out under the measuring conditions reported below in Table 1. Following a one-minute loaded phase at a γ deformation of 3 and a subsequent unloaded phase at a γ deformation of 0.003 a 90% drop in complex viscosity was recorded. Viscosity regeneration was 27% after just one minute.

### 1.2 Determining the influence of calcium chloride on hydrogel complex viscosity

Hydrogels prepared according to Example 1 with different calcium chloride fractions were rheologically characterized by oscillatory rheometry. The complex viscosity values listed below in Table 1 were obtained for the hydrogel:

**Table 1: Determination of complex viscosity of hydrogel prepared to**

| **Calcium chloride [wt%]** | **Complex viscosity η** |
|---|---|
| 0.3 | 2.18 |
| 0.1 | 23.9 |
| 0.05 | 21.2 (20.21) |
| 0.01 | 2.26 |
| 0 | 2.13 |
| Measurement settings: 22°C, value at 37°C between parentheses, deformation γ = 0.003, frequency = 10 Hz, delay time = 2 s, integration periods = 40, integration points = 2048, integration time = 1 s; the measuring system corresponds to that described above | |

### Example 1

It is clear from Table 1 that particularly high viscosities are obtainable at a calcium chloride content between 0.05 and 0.1 wt%, based on overall hydrogel weight.

A PVA hydrogel prepared to Example 1 but without gellan or calcium chloride displayed a complex viscosity drop from 2.79 Pas to 1.68 Pas on heating from 22°C to 37°C. This corresponds to a 40% drop in viscosity. By contrast, the PVA hydrogel prepared as per Example 1 with a calcium chloride content of 0.05 wt%, based on overall hydrogel weight, merely displayed a 5% viscosity drop under comparable conditions (cf. also the third entry in the table).

An amplitude sweep at 10 Hz recorded an intersection for the two G' and G" moduli at γ = 0.3. An elasticity modulus G' of 1500 Pa and a loss modulus G" of 850 Pa were determined (at 22°C) under the measuring conditions of Table 1.

### Example 2: Preparing a further thixotropic hydrogel

A reaction pot in an RT50 vacuum mix processing rig with Biotrona BA-C80 and a PX-RW 0.55 anchor stirrer from Kinematica (Switzerland) was charged with 24.3 kg of twice-distilled water from B. Braun (Eco-bag^{®}) and 2.50 kg of polyvinyl alcohol (having an average molecular weight of 100 to 300 kD (kilodaltons) and a degree of acetylation < 2%) with the trade name Mowiol^{®} 56-98 from Kuraray and sealed. This is followed by stirring the mixture at 70 to 90°C for two hours. The resulting still hot solution was then admixed with 3.00 kg of 1,2-propanediol according to EP from Merck, 100 g of sodium carboxymethylcellulose according to EP with the trade name Cekol 2000P (FIN) from Kelco, 105 g of deacetylated gellan according to EP (degree of acetylation < 4%) with the trade name KELCOGEL, 50 g of a 30 wt% solution of calcium chloride p.a. from Merck in the abovementioned twice-distilled water and also 3.75 g of FD&C Green No. 3 from Goldmann. The mixture was stirred for a further hour. Thereafter, the solution at a mixture temperature of at least 60°C was transferred into syringes using a Netzsch metering pump. The hot hydrogel did not have to be filtered. The filled syringes were sterilized in an appropriate apparatus within steam-permeable secondary packaging in a steam autoclave at 121 °C for at least 30 minutes and at most 60 minutes, preferably for 60 minutes.

Measuring the complex viscosity of the resultant PVA hydrogel with 1,2-propanediol as cryogelling control agent gave the values listed below in Table 2:

**Table 2: Determination of complex viscosity**

| **Complex viscosity η** (before freezing) | | **Complex viscosity η** (after freezing) | |
|---|---|---|---|
| Measuring tem p.=22 °C | Measuring tem p.=37 °C | Measuring temp.=22°C | Measuring temp.=37°C |
| 5.32 | 7.45 | 4.45 | 4.1 |
| Measurement settings: 22°C, value at 37°C between parentheses, deformation γ = 0.003, frequency = 10 Hz, delay time = 2 s, integration periods = 40, integration points = 2048, integration time = 1 s; the measuring system corresponds to that described under Example 1. Freezing was carried out for 5 hours in a deep freeze at -20 °C. | | | |

Table 2 does show that the addition of 1,2-propanediol results in a reduction in complex viscosity. However, the gel still had a sufficient degree of thixotropic properties after thawing. More particularly, the gel after thawing to room temperature was an elastic gel without high viscosity. An explanation for this phenomenon is believed to be that the 1,2-propanediol becomes lodged between the PVA molecules and that this form of intercalation very likely leads to a change in the hydrogen-bond structure in the PVA gel, resulting in altogether smaller PVA cells and hence smaller crystals of ice in the hydrogel.

### Example 3: Preparing a thixotropic hydrogel as two-part system

### 3.1. Preparing part A

A reaction pot in an EL10 process mixer from Escolab (Switzerland) was charged with 3.17 kg of twice-distilled water from B. Braun (Ecobag^{®}) and 292 g of polyvinyl alcohol (average molecular weight 125 kD and a degree of acetylation < 2%) with the trade name Mowiol^{®} 56-98 from Kuraray and sealed. The mixture was subsequently stirred at 70 to 90°C for two hours. Thereafter, the resulting still hot solution was admixed with 11.7 g of sodium carboxymethylcellulose according to EP with the trade name Cekol 2000P (FIN) from Kelco and 24.6 g of deacetylated gellan according to EP (degree of acetylation < 4%) with the trade name KELCOGEL^{®} and also 876 mg of FD&C Green No.3 from Goldmann. The mixture was stirred for a further hour. The solution was finally transferred into syringes using a Netzsch metering pump at a mixture temperature of at least 60°C. The hot hydrogel required no filtration.

### 3.2. Preparing part B

A reaction pot in an EL10 process mixer from Escolab (Switzerland) was charged with 3.19 kg of twice-distilled water from B. Braun (Ecobag^{®}) and 292 g of polyvinyl alcohol (average molecular weight 100 to 300 kD and a degree of acetylation < 2%) with the trade name Mowiol^{®} 56-98 from Kuraray and sealed. The mixture was subsequently stirred at 40°C for two hours. Thereafter, the resulting still hot solution was admixed with 11.7 g of sodium carboxymethylcellulose according to EP with the trade name Cekol 2000P (FIN) from Kelco and also 11.7 g of a 30 wt% calcium chloride solution (calcium chloride ≥ 98% from Merck). The mixture was stirred for a further hour and then cooled down to room temperature. The solution was then transferred into syringes using a Netzsch metering pump. Hot filtration of the hydrogel was not required.

The two filled syringes were sterilized at 121 °C in an appropriate apparatus within steam-permeable secondary packaging in a steam autoclave for at least 30 minutes and at most 60 minutes, preferably for 60 minutes.

A Gemini 150 plate-cone Bohlin rheometer was used to determine complex viscosities of hydrogels obtained on mixing parts A and B. The rheometer plate had a diameter of 40 mm and a cone angle of 4°. Gap size was 150 μm. Parts A and B were combined in a static mixing system from Medmix. The concentrations given below in Table 3 relate to the gels obtained on mixing parts A and B.

**Table 3: Determination of complex viscosities**

| **Calcium chloride [wt%]** | **Complex viscosity η** |
|---|---|
| 1 | 10.4 (7.82) |
| 0.7 | 11.3 (8.44) |
| 0.5 | 12.8 (6.92) |
| 0.1 | 8.19 (6.58) |
| 0 | 2.13 (1.08) |
| Measurement settings: 22°C, value at 37°C between parentheses, deformation γ = 0.003, frequency = 10 Hz, delay time = 2 s, integration periods = 40, integration points = 2048, integration time = 1 s; the measuring system corresponds to that described above. | |

The gels obtained in situ had a lower viscosity and were very difficult to mix, and hence inhomogeneous, because of appreciable differences in the viscosity between the gellan-containing part A and the calcium-chloride-containing part B. The drop in complex viscosity on heating from 22°C to 37°C amounted to 25 to 49% of the initial value. The pronounced decrease at low calcium chloride concentrations was conspicuous. The gels were conspicuously less elastic than the gels prepared as per Examples 1 and 2.

An elasticity modulus G' of 500 Pa and an only slightly smaller loss modulus G" of 450 Pa were determined at 22°C under the conditions reported in Table 3. The gel point on frequency sweeping in the deformation mode γ = 0.003 was 9 Hz. An amplitude sweep at 10 Hz recorded an intersection for the two G' and G" moduli at γ = 0.03.

The gel formed was significantly less stable than the gel prepared to Example 1 and also tended to transform into a sol even under small loads, so that, after administration, dislocations of the gel in the body of a patient are likely.

### Example 4: Evidencing gellan gel permeability to ions and water

A commercially available Visking^{®} dialysis tube of cellulose with a cut-off of 10 to 20 kD and a pore diameter of 1500 to 2000 ppm was used. The pore size of the tube allows the passage of water molecules and hydrated calcium ions (ion radius: 420 pm) through the dialysis tube. The components gellan, polyvinyl alcohol and sodium carboxymethylcellulose, by contrast, were unable to pass through the dialysis tube because of their larger diameters.

Before use, the dialysis tube was swelled in water for one hour. Thereafter, six pieces of tube 15 cm in length were each filled with 15 g of gel and sealed watertight at both ends with two clamps in each case. Thereafter, a dialysis was carried out against ultrapure water from a Milli-Q^{®} system. The pieces of tube were then transferred to a separate 500 ml twist-top glass containing 200 ml of ultrapure water. These glasses were subsequently placed in a shaking water bath temperature controlled to 37°C.

A sample was taken right at the beginning. Further samples were taken after 5 hours, one day, two days, five days and seven days. The weight of the dialysis liquid and of the gel was recorded for each sampling time. The calcium concentrations in the tubes were determined using ICP-OES analysis. The method had a detection limit of 5 ppm Ca²⁺.

Determination of the calcium ion content gave the values listed in the following Table 4:

**Table 4: Determination of calcium ion content**

| **Sampling time** | **Ca²⁺ in tube in ppm** | **Gel weight in tube in g** |
|---|---|---|
| 0 | 333 | 15 |
| 5 hours | 176 | 16 |
| 1 day | 169 | 20.6 |
| 2 days | 151 | 24.4 |
| 5 days | 124 | 30.3 |
| 7 days | 86 | 40 |

It is clear from Table 4 that the calcium ion concentration in the tube had decreased significantly after just five hours. This reduction could not be explained solely by the dilution effect, since the calcium concentration decreased disproportionately as a reciprocal of the dilution. Therefore, a corresponding amount of calcium ions must have already passed through the dialysis tube by this point in time. Altogether, the particulars in Table 4 show that the calcium concentration within the dialysis tube had decreased disproportionately.

## Claims

1. Thixotropic composition, preferably thixotropic hydrogel, comprising a film-forming polymer, gellan and/or agar, a physiologically compatible metal salt and water, **characterized in that** the composition includes gellan and/or agar between 0.2 and 0.4 wt%, based on overall composition weight.

2. Thixotropic composition according to claim 1, **characterized in that** the composition includes the film-forming polymer at between 0.1 and 30 wt%, especially 1 and 15 wt% and preferably 5 and 10 wt%, based on overall composition weight.

3. Thixotropic composition according to claim 1 or 2, **characterized in that** the film-forming polymer is polyvinyl alcohol.

4. Thixotropic composition according to claim 3, **characterized in that** the polyvinyl alcohol has an average molecular weight (Mp) between 100 000 and 400 000 daltons, especially 100 000 and 300 000 daltons and preferably 100 000 and 150 000 daltons.

5. Thixotropic composition according to any preceding claim, **characterized in that** the gellan and/or agar, preferably gellan, has an average molecular weight (Mp) between 100 000 and 1 000 000 daltons, especially 200 000 and 700 000 daltons and preferably 400 000 and 600 000 daltons.

6. Thixotropic composition according to any preceding claim, **characterized in that** the physiologically compatible metal salt is an inorganic metal salt, especially a salt of a divalent metal, preferably an alkaline earth metal salt, more preferably an alkaline earth metal halide and even more preferably calcium chloride.

7. Thixotropic composition according to any preceding claim, **characterized in that** the composition has a larger gellan and/or agar content, preferably gellan content, than metal salt content.

8. Thixotropic composition according to any preceding claim, **characterized in that** the proportion of metal salt in the composition is between 0.01 and 1.0 wt%, especially 0.01 and 0.1 wt% and preferably 0.02 and 0.07 wt%, based on overall composition weight.

9. Thixotropic composition according to any preceding claim, charact erized in that the composition includes water at between 50 and 98 wt%, especially 60 and 98 wt% and preferably 65 and 98 wt%, based on overall composition weight.

10. Thixotropic composition according to any preceding claim, **characterized in that** the composition further includes a tissue adhesive, preferably selected from the group consisting of sodium carboxymethylcellulose, methylpropylcellulose, hydroxyethylcellulose, hydroxypropylcellulose, cellulose sulphates, dextrins, acetylstarch, starch phosphates, carboxymethylstarch, hydroxyethylstarch, hydroxypropylstarch, salts thereof and mixtures thereof.

11. Thixotropic composition according to claim 10, **characterized in that** the tissue adhesive is present in the composition at between 0.01 and 0.49 wt%, especially 0.1 and 0.4 wt% and preferably 0.3 and 0.4 wt%, based on overall composition weight.

12. Thixotropic composition according to any preceding claim, **characterized in that** the composition further includes an anti-freeze, especially isopropanol and/or propanediol.

13. Thixotropic composition according to any preceding claim, **characterized in that** the composition is composed of the following components:
a) 0.1 to 30 wt% of film-forming polymer, preferably polyvinyl alcohol,
b) 0.2 to 0.4 wt% of gellan and/or agar, preferably gellan,
c) 0.01 to 1 wt% of physiologically compatible metal salt,
d) 0.01 to 0.49 wt% of a tissue adhesive,
e) 50 to 98 wt% of water, and optionally
f) 1 to 20 wt% of one or more admixture agents, especially to inhibit cryogelling of the composition.

14. Process for preparing a thixotropic composition, preferably a thixotropic hydrogel, according to any of claims 1 to 13, wherein a film-forming polymer, gellan and/or agar, a physiologically compatible metal salt, water and optionally one or more admixture agents are mixed with one another.

## Patentansprüche

1. Thixotrope Zusammensetzung, vorzugsweise thixotropes Hydrogel, umfassend ein filmbildendes Polymer, Gellan und/oder Agar, ein physiologisch verträgliches Metallsalz und Wasser, **dadurch gekennzeichnet, dass** die Zusammensetzung einen Anteil an Gellan und/oder Agar zwischen 0,2 und 0,4 Gew.-%, bezogen auf das Gesamtgewicht der Zusammensetzung, aufweist.

2. Thixotrope Zusammensetzung nach Anspruch 1, **dadurch gekennzeichnet, dass** die Zusammensetzung einen Anteil an dem filmbildenden Polymer zwischen 0,1 und 30 Gew.-%, insbesondere 1 und 15 Gew.-%, bevorzugt 5 und 10 Gew.-%, bezogen auf das Gesamtgewicht der Zusammensetzung, aufweist.

3. Thixotrope Zusammensetzung nach Anspruch 1 oder 2, **dadurch gekennzeichnet, dass** es sich bei dem filmbildenden Polymer um Polyvinylalkohol handelt.

4. Thixotrope Zusammensetzung nach Anspruch 3, **dadurch gekennzeichnet, dass** der Polyvinylalkohol ein mittleres Molekulargewicht (Mₚ) zwischen 100000 und 400000 Dalton, insbesondere 100000 und 300000 Dalton, vorzugsweise 100000 und 150000 Dalton, aufweist.

5. Thixotrope Zusammensetzung nach einem der vorhergehenden Ansprüche, **dadurch gekennzeichnet, dass** das Gellan und/oder Agar, bevorzugt Gellan, ein mittleres Molekulargewicht (Mₚ) zwischen 100000 und 1000000 Dalton, insbesondere 200000 und 700000 Dalton, bevorzugt 400000 und 600000 Dalton, aufweist.

6. Thixotrope Zusammensetzung nach einem der vorhergehenden Ansprüche, **dadurch gekennzeichnet, dass** es sich bei dem physiologisch verträglichen Metallsalz um ein anorganisches Metallsalz, insbesondere um ein Salz eines zweiwertigen Metalls, bevorzugt ein Erdalkalimetallsalz, weiter bevorzugt ein Erdalkalimetallhalogenid, besonders bevorzugt Calciumchlorid, handelt.

7. Thixotrope Zusammensetzung nach einem der vorhergehenden Ansprüche, **dadurch gekennzeichnet, dass** die Zusammensetzung einen größeren Gellan- und/oder Agaranteil, bevorzugt Gellananteil, als Metallsalzanteil aufweist.

8. Thixotrope Zusammensetzung nach einem der vorhergehenden Ansprüche, **dadurch gekennzeichnet, dass** der Anteil des Metallsalzes in der Zusammensetzung zwischen 0,01 und 1,0 Gew.-%, insbesondere 0,01 und 0,1 Gew.-%, bevorzugt 0,02 und 0,07 Gew.-%, bezogen auf das Gesamtgewicht der Zusammensetzung, liegt.

9. Thixotrope Zusammensetzung nach einem der vorhergehenden Ansprüche, **dadurch gekennzeichnet, dass** die Zusammensetzung einen Anteil an Wasser zwischen 50 und 98 Gew.-%, insbesondere 60 und 98 Gew.-%, vorzugsweise 65 und 98 Gew.-%, bezogen auf das Gesamtgewicht der Zusammensetzung, aufweist.

10. Thixotrope Zusammensetzung nach einem der vorhergehenden Ansprüche, **dadurch gekennzeichnet, dass** die Zusammensetzung ferner ein Gewebeadhäsionsmittel, vorzugsweise ausgewählt aus der Gruppe bestehend aus Natriumcarboxymethylcellulose, Methylpropylcellulose, Hydroxyethylcellulose, Hydroxypropylcellulose, Cellulosesulfate, Dextrine, Acetylstärke, Stärkephosphate, Carboxymethylstärke, Hydroxyethylstärke, Hydroxypropylstärke, Salze davon und Mischungen davon, aufweist.

11. Thixotrope Zusammensetzung nach Anspruch 10, **dadurch gekennzeichnet, dass** das Gewebeadhäsionsmittel einen Anteil in der Zusammensetzung zwischen 0,01 und 0,49 Gew.-%, insbesondere 0,1 und 0,4 Gew.-%, bevorzugt 0,3 und 0,4 Gew.-%, bezogen auf das Gesamtgewicht der Zusammensetzung, aufweist.

12. Thixotrope Zusammensetzung nach einem der vorhergehenden Ansprüche, **dadurch gekennzeichnet, dass** die Zusammensetzung ferner ein Gefrierschutzmittel, insbesondere Isopropanol und/oder Propandiol, aufweist.

13. Thixotrope Zusammensetzung nach einem der vorhergehenden Ansprüche, **dadurch gekennzeichnet, dass** die Zusammensetzung aus den folgenden Komponenten zusammengesetzt ist:
a) 0,1 bis 30 Gew.-% filmbildendes Polymer, bevorzugt Polyvinylalkohol,
b) 0,2 bis 0,4 Gew.-% Gellan und/oder Agar, bevorzugt Gellan,
c) 0,01 bis 1 Gew.-% physiologisch verträgliches Metallsalz,
d) 0,01 bis 0,49 Gew.-% eines Gewebeadhäsionsmittels,
e) 50 bis 98 Gew.-% Wasser und gegebenenfalls
f) 1 bis 20 Gew.-% eines oder mehrerer Zusatzstoffe, insbesondere zur Verhinderung einer Kryogelierung der Zusammensetzung.

14. Verfahren zur Herstellung einer thixotropen Zusammensetzung, vorzugsweise eines thixotropen Hydrogels, nach einem der Ansprüche 1 bis 13, bei welchem ein filmbildendes Polymer, Gellan und/oder Agar, ein physiologisch verträgliches Metallsalz, Wasser und gegebenenfalls ein oder mehrere Zusatzstoffe miteinander vermischt werden.

## Revendications

1. Composition thixotrope, de préférence hydrogel thixotrope, comprenant un polymère filmogène, du gellane et/ou de la gélose, un sel métallique physiologiquement compatible et de l'eau, **caractérisée en ce que** la composition comporte du gellane et/ou de la gélose à une teneur entre 0,2 et 0,4 % en poids, rapporté au poids total de la composition.

2. Composition thixotrope selon la revendication 1, **caractérisée en ce que** la composition comporte le polymère filmogène à une teneur entre 0,1 à 30 % en poids, en particulier 1 et 15 % en poids et de préférence 5 et 10 % en poids, rapporté au poids total de la composition.

3. Composition thixotrope selon la revendication 1 ou 2, **caractérisée en ce que** le polymère filmogène est un alcool polyvinylique.

4. Composition thixotrope selon la revendication 3, **caractérisée en ce que** l'alcool polyvinylique a un poids moléculaire moyen (Mₚ) entre 100 000 et 400 000 daltons, en particulier 100 000 et 300 000 daltons et de préférence 100 000 et 150 000 daltons.

5. Composition thixotrope selon une quelconque revendication précédente, **caractérisée en ce que** le gellane et/ou la gélose, de préférence le gellane, ont un poids moléculaire moyen (Mₚ) entre 100 000 et 1 000 000 daltons, en particulier 200 000 et 700 000 daltons et de préférence 400 000 et 600 000 daltons.

6. Composition thixotrope selon une quelconque revendication précédente, **caractérisée en ce que** le sel métallique physiologiquement compatible est un sel métallique inorganique, en particulier un sel d'un métal divalent, de préférence un sel de métal alcalino-terreux, mieux un halogénure de métal alcalino-terreux et mieux encore le chlorure de calcium.

7. Composition thixotrope selon une quelconque revendication précédente, **caractérisée en ce que** la composition a une teneur en gellane et/ou gélose, de préférence une teneur en gellane, supérieure à la teneur en sel métallique.

8. Composition thixotrope selon une quelconque revendication précédente, **caractérisée en ce que** la proportion de sel métallique dans la composition se situe entre 0,01 et 1,0 % en poids, en particulier 0,01 et 0,1 % en poids et de préférence 0,02 et 0,07 % en poids, rapporté au poids total de la composition.

9. Composition thixotrope selon une quelconque revendication précédente, **caractérisée en ce que** la composition comporte de l'eau à une teneur entre 50 et 98 % en poids, en particulier 60 et 98 % en poids et de préférence 65 et 98 % en poids, rapporté au poids total de la composition.

10. Composition thixotrope selon une quelconque revendication précédente, **caractérisée en ce que** la composition comporte en outre un adhésif tissulaire, de préférence choisi dans le groupe constitué par la carboxyméthylcellulose sodique, la méthylpropylcellulose, l'hydroxyéthylcellulose, l'hydroxypropylcellulose, les sulfates de cellulose, les dextrines, l'amidon acétylé, les phosphates d'amidon, l'amidon carboxylméthylé, l'amidon hydroxyéthylé, l'amidon hydroxypropylé, les sels de ceux-ci et les mélanges de ceux-ci.

11. Composition thixotrope selon la revendication 10, **caractérisée en ce que** l'adhésif tissulaire est présent dans la composition à une teneur entre 0,01 et 0,49 % en poids, en particulier 0,1 et 0,4 % en poids et de préférence 0,3 et 0,4 % en poids, rapporté au poids total de la composition.

12. Composition thixotrope selon une quelconque revendication précédente, **caractérisée en ce que** la composition comporte en outre un antigel, en particulier de l'isopropanol et/ou du propanediol.

13. Composition thixotrope selon une quelconque revendication précédente, **caractérisée en ce que** la composition est composée des constituants suivants :
a) 0,1 à 30 % en poids de polymère filmogène, de préférence d'alcool polyvinylique,
b) 0,2 à 0,4 % en poids de gellane et/ou de gélose, de préférence de gellane,
c) 0,01 à 1 % en poids de sel métallique physiologiquement compatible,
d) 0,01 à 0,49 % en poids d'un adhésif tissulaire,
e) 50 à 98 % en poids d'eau, et éventuellement
f) 1 à 20 % en poids d'un ou plusieurs adjuvants, en particulier pour inhiber la cryogélification de la composition.

14. Procédé de préparation d'une composition thixotrope, de préférence d'un hydrogel thixotrope, selon l'une quelconque des revendications 1 à 13, dans lequel un polymère filmogène, du gellane et/ou de la gélose, un sel métallique physiologiquement compatible, de l'eau et éventuellement un ou plusieurs adjuvants sont mélangés les uns avec les autres.
